# EUROPEAN PATENT SPECIFICATION

(11) **EP 0 506 377 B1**
(45) Date of publication and mention of the grant of the patent: **10.12.1997**
(21) Application number: 92302591.0
(22) Date of filing: 25.03.1992
(51) Int. Cl.: C07K 14/47

(54) **Calmodulin binding protein**
Calmodulin bindendes Protein
Protéine liant le calmodulin

(30) Priority: 29.03.1991 JP 89106/91; 27.12.1991 JP 358040/91
(43) Date of publication of application: 30.09.1992
(73) Proprietor: Sobue, Kenji, Ibaraki-shi, Osaka-fu (JP); TAKARA SHUZO CO. LTD., Fushimi-ku Kyoto 612 (JP)
(72) Inventor: Hayashi, Ken'Ichiro, Takatsuki-shi, Osaka-fu (JP); Asada, Kiyozo, Koga-gun, Shiga-ken (JP); Hashida, Takashi, Otsu-shi, Shiga-ken (JP); Kotani, Hirokazu, Moriyama-shi, Shiga-ken (JP); Kato, Ikunoshin, Uji-shi, Kyoto-fu (JP); Sobue, Kenji, Ibaraki-shi, Osaka-fu (JP)
(74) Representative: Marlow, Nicholas Simon

(56) References cited:
- JOURNAL OF BIOLOGICAL CHEMISTRY. (MICROFILMS) vol. 262, no. 6, 25 February 1987, BALTIMORE, MD US pages 2757 - 2763 T. FUJII ET AL. 'Domain Mapping of Chicken Gizzard Caldesmon'
- FEBS LETTERS vol. 218, no. 2, June 1987, AMSTERDAM NL pages 292 - 294 M.A. GLUKHOVA ET AL. 'Immunoreactive forms of caldesmon in cultivated human vascular smooth muscle cells'
- J. CELL. BIOL. 111 (5 PART 2), 163A, ABSTRACT NO. 894, 1990, NEW YORK, US R.E.NOVY ET AL. 'ISOLATION OF A COMPLEMENTARY DNA ENCODING A LOW M-GAMMA HUMAN CALDESMON ISOFORM'
- JOURNAL OF BIOLOGICAL CHEMISTRY. (MICROFILMS) vol. 266, no. 25, 5 September 1991, BALTIMORE, MD US pages 16917 - 16924 R.E. NOVY ET AL. 'Characterization of cDNA Clones Encoding a Human Fibroblast Caldesmon Isoform and Analysis of Caldesmon Expression in Normal and Transformed Cells'
- GENE vol. 112, 15 March 1992, AMSTERDAM NL pages 197 - 204 M.B. HUMPHREY ET AL. 'Cloning of cDNAs encoding human caldesmons'

## Description

### Field of industrial application:

This invention relates to a polypeptide that has the amino acid sequence of the functional domain of human caldesmon.

### State of the prior art:

Caldesmon is a protein that can bind with calmodulin, actin and tropomyosin, found in all tissues except skeletal muscle and cardiac muscle; it contributes to the regulation of actomyosin system in smooth muscle. This regulatory function depends on the concentration of calcium ions (flip-flop regulation) in bringing about the regulation of the actomyosin system (Proc. Natl. Acad. Sci. USA, 78, 5652-5655, 1981). When the concentration of calcium ions is low, caldesmon binds with the actin filament-tropomyosin system, and inhibits the mutual interactions of actin and myosin. When the calcium ion concentration is increased, complexes of active calmodulin and caldesmon are formed. This complex is released from the actin filament-tropomyosin system. Because of this, the inhibition of actin-myosin interaction caused by caldesmon cease, and these interactions begin.

Caldesmon was first isolated from the smooth muscles of chicken gizzards, and it has been isolated from other vertebrates. The results of the limited digestion of chicken caldesmons by the protease α-chymotrypsin have shown that a polypeptide of the size of about 35 kDa at the carboxy-terminal end of chicken caldesmons is the functional unit that brings about flip-flop regulation that depends on the concentration of calcium ions (J. Biochem., 102, 1065-1073, 1987).

There are two isoforms of chicken caldesmon that have different molecular weights. The results of electrophoresis on sodium dodecyl sulfate-polyacrylamide gels have shown that one is of high molecular weight (120,000 to 150,000Da), now named h-caldesmon, and that the other is of low molecular weight (70,000 to 80,000Da), now named l-caldesmon. h-caldesmon is abundant in smooth-muscle tissue, and l-caldesmon is abundant in non-muscle tissue and cultured cells. The amino acid sequence of h-caldesmon and the DNA sequence that codes for it have recently been identified (Biochem. Biophys. Res. Commun [called BBRC below], 164, 503-511, 1989), as have been the amino acid sequence of l-caldesmon and the DNA sequence that codes for it (J. Biol. Chem., 266, 355-361, 1991; also Japanese Patent Application 2-37362).

Both h- and l-caldesmons are of chicken origin, and the amino acid sequence of human caldesmon is still unknown.

Human platelet caldesmon₇₇ has been purified and used to prepare antibodies to use in the domain mapping of chicken gizzard caldesmon, Journal of Biological Chemistry, 262, No. 6, 25 February 1987, 2757-2763. cDNA clones spanning a coding region of 461 amino acids of low Mγ human caldesmon have been obtained, J. CELL BIOL. 111 (5 Part 2), 163A, Abstract No 894, 1990. A cDNA probe encoding the C-terminus of avian caldesmon has been used to screen a human aorta library and clone smooth-muscle and non-muscle caldesmon-encoding cDNAs. The predictions from this are that the smooth-muscle protein is 793 aa long and the non-muscle protein, missing the central helical domain of 256 aa, is 537 aa long, Gene 112 (1992) 197-204.

The Journal of Biological Chemistry Vol. 226, No. 25, September 5th 1991, pp 16917-24 discloses the characterisation of cDNA clones encoding a human fibroblast caldesmon isoform which span the entire coding region (538 amino acids). Human, rat and chicken 1-CaD amino acid sequences are compared.

The morphological change of cells is one characteristic of carcinogenesis. The preservation of cell morphology is closely related to the actin network of the cells, and when cells are transformed, the change in cell morphology occurs in correlation with loss of actin cables, which are part of the filament system that makes up the cytoskeleton (Proc. Natl. Acad. Sci. USA, 72, 994-998, 1975). Immunohistochemical analysis have revealed that caldesmon is localized in actin cables, cell attachment sites, and membrane ruffles in normal cells, but in cancer cells, the compound is not found in any defined cell location (Proc. Natl. Acad. Sci. 81, 3133-3137, 1984).

When cultured cells are transformed with a variety of oncogenic viruses, the caldesmon concentration of the cells decreases and the phosphorylation of the caldesmon increases (Proc. Natl. Acad. Sci. USA 81, 3133-3137, 1984, and Saibo Kogaku [Cell Technology], suppl. 3, "Molecules regulating calcium signalling," 140-153, 1987).

### Problems to be solved by this invention

As described above, there seems to be a close relationship between caldesmon content and the change in morphology of cells that have been transformed. If the relationship were understood, it might be possible to use human caldesmon for treatment of cancer. In addition, if the DNA sequence that codes for human caldesmon were known, it would be possible to use it for the diagnosis of cancer. Further, if it were possible to isolate a polypeptide that has activities of the actin-binding functional unit, the tropomyosin-binding functional unit, and the calmodulin-binding unit, this polypeptide could be used to inhibit or accelerate the mutual interactions between actin and myosin whatever the calcium concentration is, so that the polypeptide could be used as a vasodilator or a regulator of the movements of the digestive tract.

The purpose of this invention is to provide a polypeptide that has the activity of human caldesmon and the DNA sequence that codes for said polypeptide.

### Steps taken to solve the problem.

To summarize this invention, the first part of this invention relates to a polypeptide that has a calmodulin-binding activity and an actin-binding activity characterized by said polypeptide has an amino acid sequence of sequence ID No.1 in the sequence listing in a polypeptide molecule. The second part of this invention relates to a gene that codes for the polypeptide of the first part of this invention.

The polypeptide of this invention is a polypeptide that has both calmodulin-binding activity and actin-binding activity; it is, for example, a polypeptide that has within it the amino acid sequence shown as sequence ID No.1 in the sequence listing, which sequence is that of the functional unit of human caldesmon, which unit was discovered by the inventors of this invention. Polypeptides that have within them this amino acid sequence include, for example, the polypeptides that have the sequence ID No.2 to 5 of the sequence listing.

A gene that codes for the polypeptide of artificial human caldesmon is, for example, a gene with the DNA sequence of sequence ID No.7 to 12 in the sequence listing, and genes that can hybridize to the gene with the sequence ID No.7 to 12, and which genes code for a polypeptide that has calmodulin-binding activity and actin-binding activity.

The inventors of this invention prepared a cDNA library from HeLa cells, a human cell line, and next selected cDNA clones that code for a polypeptide of human caldesmon from this cDNA library; the DNA sequence and the amino acid sequence coded for human caldesmon were deduced by DNA sequence analysis. Next, based on various finding, the inventors prepared polypeptides of various lengths, and looked for the functional domain of human caldesmon polypeptide, using calmodulin-binding activity and actin-binding activity as indices. Based on the findings described below, the inventors achieved this invention.

Below, this invention is explained in detail.

The gene that codes for human caldesmon can be isolated and analyzed as described here. To obtain the gene of human caldesmon, HeLa cells or the like can be used, and the total RNA, which includes the poly(A)⁺ RNA, of the cells, was isolated, and bound onto a cellulose carrier or the like with oligo(dT). This was used as a template in the synthesis of cDNA with reverse transcriptase. The cDNA was synthesized by the method of Okayama-Berg or by the method of Gubler-Hofmann. The cDNA synthesized in this was ligated with a plasmid or phage vector and introduced into a host, giving a cDNA library.

The cDNA library was screened for the desired clones by one of the two following methods. The amino acid sequence of a portion of the desired protein was identified, and an oligonucleotide that coded for that amino acid sequence was synthesized and used as a probe in screening. Or, an antibody that bound with the desired protein was prepared and used to screen the cDNA library constructed in an expression vector, in an immunological method.

When the immunological method of screening is used, λgtl1 is well used as the expression vector. λgt11 is commercially available; it can be purchased from, for example, Stratagene. When λgt11 is used in immunological screening, first, the library is amplified on agar plates, and nitrocellulose or nylon filters treated with isopropyl-β-D-galactopyranoside (IPTG) are placed on these plates and expression is induced, after which the protein being expressed adsorbes onto the filter. The filter is immersed in buffer that contains bovine serum albumin (below, referred to as the blocking buffer), and after this step of blocking, antibodies (first antibody) that bind with the desired protein are added to fresh blocking buffer, and the filter is immersed in this blocking buffer, so that the desired protein and the antibodies form complexes. After the filter is washed, it is immersed in a blocking buffer that contains the second antibody, labelled with an enzyme. The first antibody and the second antibody form complexes. Second antibodies coupled to alkaline phosphatase or peroxidase are commercially available. The filter is washed to remove excess second antibody that has not adsorbed to the filter, and the filter is put in a developing solution, and colored clones are selected. The recombinant phages obtained in this way are used to infect host Escherichia coli cells, and phage DNA is obtained from the phage lysate. The cDNA from the recombinant phage DNA is isolated and purified, and its DNA sequence is identified.

The antibodies used for immunological screening can be antisera prepared in rabbits immunized with other kinds of caldesmon, such as chicken caldesmon that have amino acid sequences likely to correspond in part to the sequence of the desired protein, human caldesmon.

The cDNA sequence coding for human caldesmon, which has been identified by the inventors, is sequence ID No.13 and 14 in the sequence listing. Of the two forms of human caldesmon, the 558 amino acid residues shown as sequence ID No.6 in the sequence listing is that of the caldesmon of high molecular weight, and the DNA sequence that codes for this is sequence ID No.12 of the sequence listing. The caldesmon of low molecular weight is exactly the same as that of high molecular weight except that it lacks amino acids 202 to 227, and it is shown as the 532 amino acids of sequence ID No.5 in the sequence listing, and the DNA sequence that codes for this is sequence ID No.11 in the sequence listing. Here, the form of higher molecular weight is called type I, and the form of lower molecular weight is called type II.

To obtain plasmids that express the polypeptides of human caldesmons type I and type II, DNA that codes for the caldesmon of type I and DNA that codes for the caldesmon of type II are prepared separetely from the recombinant phage DNA mentioned above, and the DNA is inserted into an expression plasmid, such as the plasmid pTV118N. The plasmid that coded for the caldesmon of type I was designated pTV118NHS3CaDl, and the plasmid that coded for the caldesmon of type II was designated pTV118NHS3CaD2.

With pTV118NHS3CaDl, it is possible to prepare DNA that codes for polypeptides of various lengths. For example, a site slightly upstream of the initiation codon of the region that codes for type I caldesmon of this plasmid can be cleaved with an appropriate restriction enzyme, and exonuclease can be used to remove the sequence of the 5'-side in the caldesmon gene. By changes in the reaction conditions, it is possible to prepare genes that code for polypeptides of various chain lengths that are missing the N-terminal portion of type I caldesmon. Alternatively, it is possible to amplify DNA of various lengths by the polymerase chain reaction (PCR: Saiki et al., Science, 230, 1350-1354, 1985) with a number of primers synthesized and pTV118NHS3CaD1 as a template, and said DNA can be used to prepare polypeptides of various lengths. As described in detail, with the sequence ID No.15 and 16 in the sequence listing as primers, PCR can be done (at 94°C for 30 sec in step 1, at 55°C for 2 min in step 2, and at 72°C for 1 min in step 3, for a total of 20 cycles), which results in the amplification of DNA that contains the DNA sequence shown as sequence ID No.10 in the sequence listing, which codes for the polypeptide shown in the sequence listing as having sequence ID No.4 (called 312AA below). With the sequence ID No.17 and 18 in the sequence listing as primers, it is possible to amplify DNA that contains the DNA sequence shown as sequence ID No.9 in the sequence listing, which codes for the polypeptide shown in the sequence listing as having sequence ID No.3 (called 122AA below). With the sequences ID No.18 and 19 in the sequence listing as primers, it is possible to amplify DNA that contains the DNA sequence shown as sequence ID No.8 in the sequence listing, which codes for the polypeptide shown in the sequence listing as having sequence ID No.2 (called 118AA below). With the sequence ID No.20 and 21 in the sequence listing as primer, it is possible to amplify DNA that contains the DNA sequence shown as sequence ID No.23 in the sequence list, which codes for the polypeptide shown in the sequence listing as having sequence ID No.22 (called 94AA below). In addition, with the sequences ID No.21 and 24 in the sequence listing as primers, it is possible to amplify DNA that contains the DNA sequence shown as sequence ID No.26 in the sequence listing, which codes for the polypeptide shown in the sequence listing as sequence ID No.25 (called 90AA below). Next, these DNAs are inserted into expression plasmids, such as, for example, pTV118N. The plasmid that coded for 312AA was named pTHCD247, the plasmid that coded for 122AA was named pTHCDXa443, the plasmid that coded for 118AA was named pTHCD443, the plasmid that coded for 94AA was named pTHCDXa451, and the plasmid that coded for 90AA was named pTHCD451.

The plasmids were used to transform Escherichia coli cells, such as E. coli JM109, for their expression, and the recombinant cells were cultured under appropriate conditions so that the desired polypeptides accumulated within the E. coli cells. The purification of the polypeptides was, for example, as follows. The recombinant E. coli cells were cultured in a culture medium such as L broth, and the cells were harvested and disrupted by being sonicated. The disrupted cells were centrifuged and the supernatant was obtained. After treatment such as dialysis, the dialysate was put on a column for gel filtration and then on a column for ion-exchange chromatography for purification. The desired proteins were purified by use of affinity columns coupled with calmodulin or tropomyosin, making use of the properties of the target protein. 122AA and 94AA have a sequence that can be recognized by factor Xa. By cleaving with factor Xa site-specifically, it is possible to prepare a polypeptide shown as sequence ID No.1 in the sequence listing (called 116AA below) and a polypeptide shown as sequence ID No.27 in the sequence listing (called 88AA below).

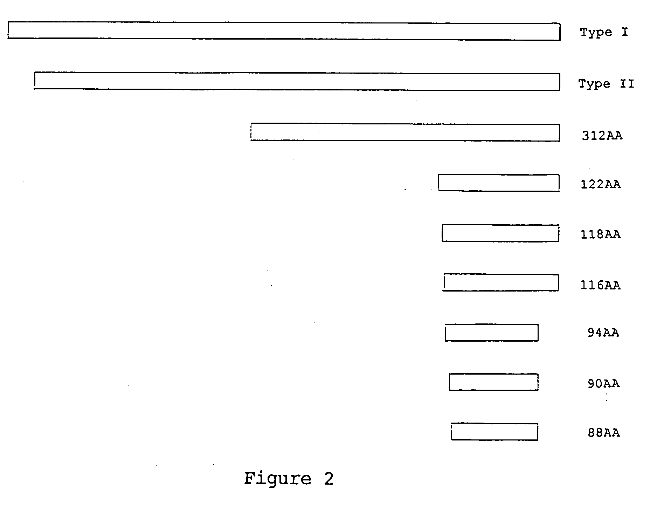

The polypeptides to which this invention is related are shown in Figure 2. Type II is a polypeptide that is missing one portion of type I. 312AA is a polypeptide that is the C-terminal portion of types I and II, 122AA and 118A are polypeptides that have a sequence of 116 amino acids in the C-terminal sequence of 312AA, and 94AA and 90AA are polypeptides that have a sequence of 88 amino acids that are in the central portion of 118AA, lacking both the N-terminal portion and the C-terminal portion. On the N-terminal of 118AA, there are residues Met-Ala that originated from a PCR primer, and on the N-terminal of 90AA, there are residues Met-Ala that also originated from a PCR primer. On the N-terminal of 122AA and 94AA, there are residues Met-Ala that originated from PCR primer and a sequence that can be recognized by factor Xa.

It is possible to measure the calmodulin-binding activity of these polypeptides by use of their affinity for calmodulin during calmodulinaffinity chromatography on a column, and it is possible to measure the actin-binding activity of these polypeptides by the method of coprecipitation with actin. As shown in Table 1, the polypeptide (typeI, typeII,312AA, 122AA, 118AA, and 116AA) that contains the polypeptide of 116 amino acids (called 116AA below) that has sequence ID No.1 in the sequence listing has both calmodulin-binding activity and actin-binding activity.

**Table 1**

| polypeptide | calmodulin-binding activity | actin-binding activity |
|---|---|---|
| typeI | + | + |
| typeII | + | + |
| 312AA | + | + |
| 122AA | + | + |
| 118AA | + | + |
| 116AA | + | + |
| 94AA | + | - |
| 90AA | + | - |
| 88AA | + | - |
| (Activity is shown as +, and lack of activity as -.) | | |

Type I, type II, 312AA, 122AA, 118AA, 116AA all have tropomyosin-binding activity and have a inhibitory activity to actomyosin ATPase, and so the functional unit of human caldesmon is identified as being 116AA.

It is possible to construct a DNA that codes for 116AA out of pTHCD443 as needed by the method of site-specific mutagenesis, and the DNA can be used to express 116AA.

As described above in detail, this invention provides various polypeptides that have activities of human caldesmon and also provides genes that code for these polypeptides. It is possible to produce both on a large scale by the use of genetic engineering, and said genes and polypeptides will be useful in diagnosis and other fields of medicine, and in biochemical research, as well. The functional unit of human caldesmon, 116AA, can be used as a material for chimera proteins and peptide transfer, and the DNA with sequence ID No.7 in the sequence listing will be of use in the fields of genetic engineering and protein engineering.

### Examples

Below, this invention will be explained in further detail with reference to examples, but this invention is not to be taken to be limited to these examples.

### Example 1.

### Cloning of cDNA that codes for the polypeptide of human caldesmon from HeLa cells

### 1-1. Construction of a cDNA library.

The total RNA of HeLa cells was obtained by the method of guanidium-cesium chloride (Biochemistry, 18, 5294-5299, 1979), and poly(A)⁺ RNA was isolated by column chromatography with oligo-(dT)cellulose. The purified poly(A)⁺ RNA was used as a template and oligo-(dT) was used as the primer in the synthesis of cDNA by the method of Gubler-Hoffmann (Gene, 25, 263-269, 1983). The ends of the cDNA were blunted with T4 DNA polymerase, and the EcoRI site in the cDNA was methylated with EcoRI methylase. Next, cDNA was ligated to an EcoRI linker [d(pGGAATTCC)] with use of T4 DNA ligase, and cDNA with both ends having an EcoRI was constructed by EcoRI digestion. This cDNA was ligated with the EcoRI arm of λgt11 (Stratagene), and a cDNA library was made by in vitro packaging with GigapackII Gold (Stratagene).

### 1-2. Screening of the cDNA library

Plaques of phages with use of E. coli Y1090 as the host cells were formed. To do this Y1090 cells were cultured overnight at 37°C in L medium that contained 0.02% maltose, and the cells were collected by centrifugation. The cells harvested were suspended in 10 mM MgSO₄ and were added into a suspension of phage solution and kept at 37°C for 15 minutes to allow attachment of the phages to the host cells. To this mixture was added a mixture of soft agar (L medium containing 10 mM MgSO₄ with agarose added to the final concentration of 0.6%, which medium was autoclaved and then cooled to and kept at 50°C), and this new mixture was spread on L plates (below, this step is referred to as "plating"). The plates were kept at 42°C for 3 hours, and after formation of plaques, a nylon membrane (Hybond-N; Amersham) treated with 100 mM IPTG was placed on the plate and the whole was kept at 37°C for 3 hours. The nylon membrane was removed from the plate and immnologically screened with rabbit anti-caldesmon antibodies obtained by the immunization of a rabbit with 35kDa fragment of chicken caldesmon obtained by the digestion, with α-chymotrypsin (J. Biochem., 102, 1065-1073, 1987) and with F(ab')₂ fragment of goat anti-rabbit immunoglobulin antibody labelled with alkaline phosphatase as the second antibody. Clones that were stained with nitro blue tetrazolium (NBT) and 5-bromo-4-chloro-3-indolylphosphate (BCIP) were positive. Several positive signals were obtained from among the 250,000 clones. The plaques that corresponded to the positive signals were cut out together with agar and suspended in 500 µl of SM solution (50 mM Tris-HCl, 100 mM NaCl, and 10 mM MgSO₄, pH 7.5), plated after being diluted appropriately, and screened again as described above, by which procedure it was possible to isolate four phage clones independently.

### 1-3. Preparation of recombinant λgtl1 DNA.

Next, 10⁵ of the cloned phages were plated, and the plates were kept first at 42 °C for 3 hours and then at 37°C overnight. After this, 15 ml of SM solution and a few drops of chloroform were added and the plates were kept at room temperature for 30 minutes. The upper layer of soft agar with the SM solution was removed and put into a centrifuge tube, and centrifuged at 3000 r.p.m for 15 minutes. The supernatant was added 40% (w/v) polyethylene glycol 6000, so that the final concentration of the polyethylene glycol was 10%, and mixed throughly. The mixture was kept at 4°C for 1 hour and centrifuged at 3000 r.p.m. for 15 minutes. The supernatant was discarded and the precipitate was suspended with SM solution and then left at 4°C (below, this method is referred to as the plate lysate method). By this procedure, a phage solution with the titer of 1 x 10¹⁰ pfu/ml was obtained.

E. coli Y1090 cells that had been cultured overnight in L medium were harvested and suspended in 10 mM MgSO₄. The cell suspension was mixed with the phage solution mentioned above at multiplicity of infection (moi) of 0.01, and the mixture were left at 37°C for 15 minutes. The mixture of phages and E. coli cells was used to inoculate L medium that contained 10 mM MgSO₄, and continued to be cultured at 37°C until a considerable amount of bacterial debris remained after lysis. To the culture, sodium chloride was added to the final concentration of 0.5 M, chloroform was added to the final concentration of 0.5%, and the mixture was stirred at 37°C for 15 minutes. The supernatant obtained by centrifugation was mixed with polyethylene glycol 6000 added to the final concentration of 10% (w/v), and the mixture was left overnight at 4°C. The phage precipitate obtained by centrifugation was dissolved in TM solution (50 mM Tris-HCl and 10 mM MgSO₄, pH 7.8), and by the method of glycerol step-gradient ultracentrifugation (T.Maniastis et al., Molecular cloning: A laboratory manual, pp. 83-84, Cold Spring Harbor Laboratory, 1982), the phages were purified.

The phages obtained were suspended in TM solution, and DNase I and RNaseA were added to the suspension, which was then kept at 37°C for 30 minutes before the addition of EDTA, Proteinase K (Sigma), and SDS to the concentrations of 20 mM 50 µg/ml, and 0.5%, respectively. After then the mixture was kept at 65°C for 1 hour. Phenol extraction was done, followed by extraction with diethyl ether, and to the aqueous layer, a 1/10 volume of 5 M sodium chloride and two volumes of ice-cold ethanol were added, which caused precipitation of the DNA (below, this step is referred to as ethanol precipitation). The mixture was centrifuged and the precipitated DNA was collected, after which it was washed in 70% ethanol and dried before being dissolved in 100 µl of TE solution (10 mM Tris-HCl and 1 mM EDTA, pH 8.0).

### 1-4. Identification of DNA sequence of the inserted fragment

The recombinant λgt11 DNA prepared as described above was digested with EcoRI, and the inserted fragments were isolated and purified. Then they were cloned at the EcoRI site of M13mp18RF DNA. The recombinant M13mp18RF DNA was digested with SalI and SphI, and by use of exonuclease III (Course in Biochemical Experimentation 1, Methods in genetic research I, pp. 186-200, 1986), a mutant was constructed that lacked various length of sequence at the 5'-side of the SalI end. Another deletion mutant was prepared that was of recombinant M13mp18RF DNA that had the fragment insertion in the opposite orientation. Then E. coli JM109 cells were transformed with each mutant DNA derived from the recombinant M13mp18 and single-stranded DNA was isolated from the M13mp18 derivatives. Its DNA sequence was identified by the dideoxy-mediated chain-termination method, and the amino acid sequence was deduced from the DNA sequence.

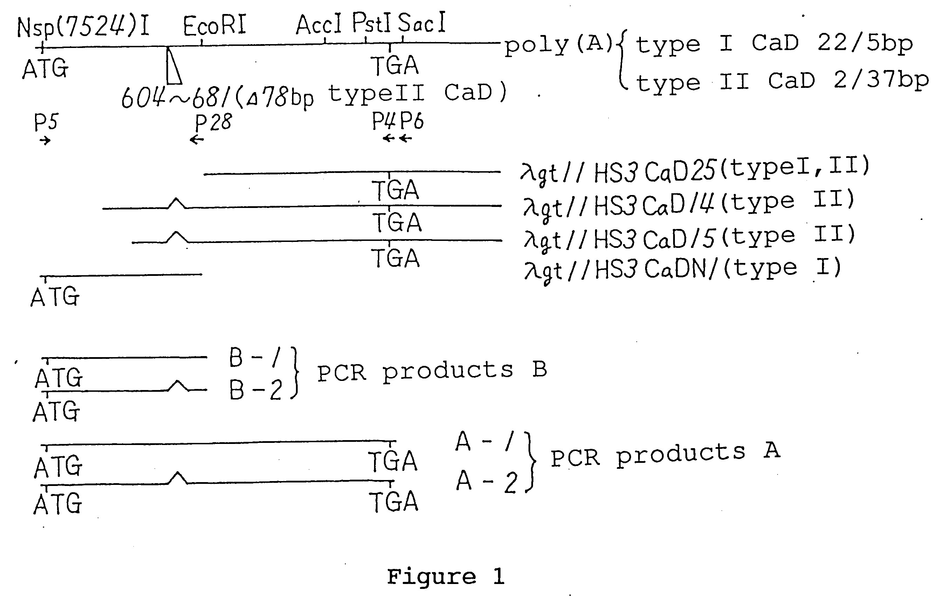

Restriction maps of these clones are shown in Fig. 1. The ATG and TGA shown in the Fig. 1 are the initiation codon and the termination codon for translation, respectively.

The results of identification of the DNA sequence suggested that HeLa cells had two kinds of caldesmon. This was checked by use of the PCR. To do this, with primer 6 (shown in Fig. 1 as P6) indicated as sequence ID No.29 in the sequence listing the first strand of cDNA was synthesized. With both primer 5 (shown in Fig. 1 as P5) indicated as sequence ID No.30 of the sequence listing and primer 4 (shown in Fig. 1 as P4) indicated as sequence ID No.31 in the sequence listing as primer 4, the PCR was done, and two kinds of PCR product A (A-1 and A-2) were obtained. Then with primer 5 and primer 28 (shown in Fig. 1 as P28) indicated as sequence ID No.32, the PCR was done, and two kinds of PCR product B (B-1 and B-2) were obtained. Restriction maps of PCR products A (A-1 and A-2) and B (B-1 and B-2) are shown in Fig. 1. The DNA sequences of these PCR products were identified by the dideoxy method, and it was confirmed that there were two kinds of caldesmon. The caldesmon of higher molecular weight was composed of the 558 amino acid residues shown as sequence ID No.6 in the sequence listing, and the caldesmon of lower molecular weight was composed of the 532 amino acid residues shown as sequence ID No.5 in the sequence listing and was exactly the same as that of higher molecular weight except that it lacked amino acid residues 202 to 227 of the larger caldesmon. The caldesmon of higher molecular weight was named type I, and the caldesmon of lower molecular weight was named type II. The DNA sequence of type I is that shown as sequence ID No.12 in the sequence listing, and the DNA sequence of type II is that shown as sequence ID No.11. Their cDNA sequences are shown as sequence ID No.14 and 13, respectively, in the sequence listing.

### Example 2. Construction of expression vectors for types I and II

### 2-1. Construction of a plasmid that coded for type I caldesmon.

A cDNA fragment of about 800 bp that coded for the N-terminal region of type I caldesmon was obtained by EcoRI digestion of λgt11HS3CaDN1, and cloned at the EcoRI site of M13mp18RF DNA, giving M13mp18HS3CaDN1. This M13mp18HS3CaDN1 was digested with Nsp(7524)I, blunt-ended with T4 DNA polymerase, and digested with EcoRI, giving cDNA fragments of about 790 bp. This cDNA fragment was cloned in plasmid pTV118N which was digested with NcoI, treated with Klenow fragment (large fragment of E. coli DNA polymerase I), and digested with EcoRI, giving pTV118NHS3CaDN1.

cDNA fragments about 1.3 kbp long that coded for the C-terminal region of caldesmon were obtained by EcoRI digestion of λgt11HS3CaD25, and cloned at the EcoRI site of M13mp18RF DNA, giving M13mp18HS3CaDC. Then M13mp18HS3CaDC was digested with EcoRI and SacI, giving fragments of cDNA about 940 bp long, which were into pTV118NHS3CaDN1 between the EcoRI and SacI sites , giving plasmid pTV118NHS3CaD1, which coded for type I caldesmon.

Cells of E. coli JM109 into which pTV118NHS3CaD1 had been introduced were designated E. coli JM109/pTV118NHS3CaD1, and deposited at the Fermentation Research Institute of the Agency of Industrial Science and Technology, Japan, under FERM BP-3673.

### 2-2. Construction of a plasmid that coded for type II caldesmon

T4 DNA polymerase was used to make blunt ends on PCR product A-2, and the product was cloned at the SmaI site of M13mp18RF DNA so as to be in the same direction in the PCR product A-2 as in the M13mp18 lacZ gene. Then cloned M13mp18PCRA-2 with the plasmid A-2 as an insertion was constructed. M13mpl8PCRA-2 was digested with Nsp(7524)I, and after its ends were made blunt with T4 DNA polymerase, it was digested at the multicloning site that originated from M13mp18 with XbaI, and cDNA fragments about 1.6 kbp long were obtained. These were cloned into pTV118N that had been digested with NcoI, treated with Klenow fragment, and then digested with XbaI, giving pTV118NHS3CaD2 PCRA-2. This was digested with EcoRI and HindIII, and ligated with the cDNA fragments about 940 bp long that code for the C-terminal region of the caldesmon obtained by digestion of pTV118NHS3CaD1 with EcoRI and HindIII. The product was designated plasmid pTV118NHS3CaD2, which codes for type II caldesmon.

E. coli cells into which pTV118NHS3CaD2 had been introduced were designated E. coli JM109/pTV118NHS3CaD2, and deposited at the Fermentation Research Institute of the Agency of Industrial Science and Technology, Japan, under FERM P-12013.

### Example 3. Construction of a plasmid that expresses human caldesmon polypeptide

### 3-1. Construction of a plasmid that codes for 312AA

A primer with sequence ID No.15 of the sequence listing and with the NcoI recognition sequence at its 5'-end and a primer with sequence ID No.16 and with the SacI recognition sequence at its 5'-end were synthesized with a DNA synthesizer and purified. Said primers were used together with pTV118NHS3CaDl as the template in the PCR to amplify DNA that had the DNA sequence shown as sequence ID No.10 in the sequence listing within its sequence.

Next, this amplification product was digested with NcoI and SacI and extracted with buffered phenol, so that its enzyme activity was lost. For the removal of an excess of dNTP in the PCR reaction mixture, and DNA concentration, the DNA was caused to precipitate with ammonium sulfate and isopropyl alcohol. The precipitated DNA was dissolved with TE solution (10 mM Tris-HCl, pH 8.0, and 1 mM EDTA), and ligated with pTV118N that had been digested with both NcoI and SacI, giving the plasmid pTHCD247, which codes for 312AA.

E. coli JM109 cells into which pTHCD247 had been introduced were designated E. coli JM109/pTHCD247, and deposited at the Fermentation Research Institute of the Agency of Industrial Science and Technology, Japan, under FERM BP-3671.

### 3-2. Construction of a plasmid that codes for 118AA

A primer with sequence ID No.19 of the sequence listing and with the NcoI recognition sequence at its 5'-end and a primer with sequence ID No.18 and with the EcoRI recognition sequence at its 5'-end were synthesized with a DNA synthesizer and purified. Said primers were used together with pTV118NHS3CaD1 as the template in the PCR to amplify DNA that had the DNA sequence shown as sequence ID No.8 in the sequence listing within its sequence.

Next, this amplification product was digested with NcoI and EcoRI, and treated as in section 3-1 above before being ligated with pTV118N that had been digested with both NcoI and EcoRI, giving the plasmid pTHCD443, which codes for 118AA.

E. coli JM109 cells into which pTHCD443 had been introduced were designated E. coli JM109/pTHCD443, and deposited at the Fermentation Research Institute of the Agency of Industrial Science and Technology, Japan, under FERM BP-3672.

### 3-3. Construction of a plasmid that codes for 122AA

A primer with sequence ID No.17 of the sequence listing and with the NcoI recognition sequence and the factor Xa recognition sequence at its 5'-end was synthesized with a DNA synthesizer and purified. Next, said primer and the primer described above as having sequence ID No.18 were used together with pTV118NHS3CaD1 as the template in the PCR to amplify DNA that had the DNA sequence shown as sequence ID No.9 in the sequence listing within its sequence.

Next, this amplification product was digested with NcoI and EcoRI, and ligated with pTV118N, which had also been digested both NcoI and EcoRI, giving a plasmid that coded for 122AA. Said plasmid was designated pTHCDXa443, and cells of E. coli JM109 into which the plasmid was introduced were named E. coli JM109/pTHCDXa443.

### 3-4. Construction of a plasmid that codes for 90AA

A primer with sequence ID No.24 of the sequence listing and with the NcoI recognition sequence at its 5'-end and a primer with sequence ID No.21 and the EcoRI recognition sequence at its 5'-end were synthesized with a DNA synthesizer and purified. Said primers were used together with pTV118NHS3CaD1 as the template in the PCR to amplify DNA that had the DNA sequence shown as sequence ID No.26 in the sequence listing within it. Next, this amplification product was digested with NcoI and EcoRI, and ligated with pTV118N that had been digested with NcoI and EcoRI, giving the plasmid pTHCD451, which codes for 90AA.

E. coli JM109 cells into which pTHCD451 had been introduced were designated E. coli JM109/pTHCD451.

### 3-5. Construction of a plasmid that codes for 94AA

A primer with sequence ID No.20 of the sequence listing and with the NcoI recognition sequence and the factor Xa recognition sequence at its 5'-end was synthesized with a DNA synthesizer and purified. Said primer and the primer described above as having sequence ID No.21 were used together with pTV118NHS3CaD1 as the template in the PCR to amplify DNA that had the DNA sequence shown as sequence ID No.23 in the sequence listing within its sequence.

Next, this amplification product was digested with NcoI and EcoRI, and ligated with pTV118N, which had also been digested both NcoI and EcoRI, giving a plasmid that coded for 94AA. Said plasmid was designated pTHCDXa451, and cells of E. coli JM109 into which the plasmid was introduced were named E. coli JM109/pTHCDXa451.

### Example 4. Expression of human caldesmon polypeptide in E. coli

### 4-1. Expression of type I in E. coli

Cells of E. coli JM109 which carried pTV118NHS3CaD1 (FERM BP-3673) were used to inoculate 5 ml of L medium that contained 50 µg/ml ampicillin, and the culture was incubated with shaking overnight at 37°C. This seed culture was used to inoculate 500 ml of L medium with the same concentration of ampicillin, and the culture was incubated with shaking overnight at 37°C. When the cells reached the mid-logarithmic stage of growth, IPTG was added to the final concentration of 2 mM, and culture was continued for 17 hours more before the cells were harvested.

The cells were suspended in E solution (0.3 M KCl, 0.5 mM EGTA, 0.5 mM MgC1₂, 0.5 mM dithiothreitol (DTT), 0.3 mM PMSF, and 50 mM Tris-HCl, pH 7.0), and the suspension was sonicated to disrupt the cells. The suspension was centrifuged and the supernatant was incubated at 95°C for 5 minutes and then cooled. This was centrifuged and the precipitate was removed. The supernatant was dialyzed against C1 solution (120 mM NaCl, 0.1 mM EGTA, 0.2 mM DTT, and 10 mM Tris-HCl, pH 7.3). The inner solution during dialysis was passed through a column of DEAE Toyopearl 650M equilibrated with C1 solution. To the pass-through fraction, calcium chloride was added to the final concentration of 5 mM, the mixture was passed through a column of Sepharose 4B (Pharmacia LKB) coupled with calmodulin which equilibrated with C2 solution (70 mM NaCl, 0.2 mM CaCl₂, 0.1 mM DTT, and 10 mM Tris-HCl, pH 7.5) as described elsewhere (Journal of Biochemistry, 102, 1065-1073, 1987), and type I purified was extracted with C2 solution that contained 1.2 mM EGTA and purified.

### 4-2. Expression of type II in E. coli

Cells of E. coli JM109 which carried pTV118NHS3CaD2 (FERM P-12013) were cultured under the same conditions as in the example of 4-1 above, extracted, heated, put on a column of resin for affinity chromatography with calmodulin, and purified, giving type II.

### 4-3. Expression of 312AA, 122AA, 118AA, 94AA, and 90AA in E. coli

Cells of E. coli JM109/pTHCD247 (FERM BP-3671), of E. coli JM109/pTHCDXa443, of E. coli JM109/pTHCD443 (FERM BP-3672), of E. coli JM109/pTHCDXa451 and of E. coli JM109/pTHCD451 were cultured under the same conditions as in the example of 4-1 above, and purification gave 312AA, 122AA, 118AA, 94AA, and 90AA.

### 4-4. Preparation of 116AA and 88AA

122AA and 94AA were treated with restriction proteinase factor Xa (Takara Shuzo Co., Ltd.) for 5 hours at 37°C, and a column of calmodulin-Sepharose was used to purify 116AA and 88AA from the digest.

### Example 5. Assay of physiological activity

### 5-1. Assay of calmodulin-binding activity and actin-binding activity

For the assay of calmodulin-binding activity, polypeptides purified as described above were mixed with 10 volumes of CaB buffer (10 mM Tris-HCl, pH 7.5, 100 mM KCl, 0.1 mM DTT, and 0.2 mM CaC1₂), and put on a column of Sepharose 4B (Pharmacia) coupled with calmodulin which equilibrated with the same buffer. This allowed binding of the polypeptide with calmodulin. After the column was washed with a large amount of the same buffer, the bound polypeptide was eluted from the column with the same buffer, except that 1 mM EGTA was added to the buffer instead of CaC1₂. A portion of each fraction was put on an SDS-polyacrylamide gel and electrophoresed, and the presence of the polypeptide in question was confirmed. For the assay of actin-binding activity, the co-precipitation method (BBRC, 132, 645-651, 1985, and Journal of Biochemistry, 102, 1065-1073, 1987) was used.

To 100µ1 of a 2 mg/ml solution of monomeric actin prepared by the Staub method (Journal of Biological Chemistry, 188, 559 (1959)), 20 µl of 10 mM ATP, 40 µl of 5x buffer (50 mM Tris-HCl, pH 7.5, 500 mM KCl, 1 mM CaC1₂, and 0.5 mM DTT), 20 µl purified polypeptide, and water were added for a total volume of 196 µl, and then 4 µl of 100 mM MgC1₂ was added. The mixture was left at room temperature for 1 hour. By the addition of MgC1₂, the monomeric actin was polymerized and became filamentous actin. After the polymerization, the mixture was centrifuged at 200,000 x g for 30 minutes. The monomeric actin remained in the supernatant, whereas the filamentous actin and proteins bound with it (the polypeptide of this invention) precipitated together. The supernatant and the precipitate were separately electrophoresed on SDS-polyacrylamide gels, and the binding of the polypeptide with actin was confirmed.

The results shown in the table 1 above, were that the polypeptide that contained 116AA in its sequence had both kinds of activity.

### 5-2. Assay of tropomyosin-binding activity and inhibition of actomyosin ATPase activity

The ability of type I, type II, 312AA, 122AA, 118AA, and 116AA which have the activities described in 5-1, to bind with tropomyosin and to inhibit actomyosin ATPase activity was measured.

For the assay of tropomyosin-binding activity, an affinity column with tropomyosin was prepared by the coupling of tropomyosin with Sepharose 4B (Pharmacia) activated with CNBr. The column was equilibrated with TMB buffer (10 mM Tris-HCl, pH 7.0, 2 mM MgC1₂, and 0.5 mM DTT), and a solution of a purified polypeptide diluted 10 times in the same buffer was put on the column to allow binding of the polypeptide to the column. The column was washed throughly with the same buffer, and after substances that had bound nonspecifically were removed in this way, elution was done with slowly increasing concentrations of potassium chloride. A portion of each fraction was put on an SDS-polyacrylamide gel and electrophoresed, and the polypeptide in question was found in the fractions that had been eluted with potassium chloride at the concentrations of 40 to 60 mM.

For the assay of the inhibition of actomyosin-ATPase by the polypeptide of this invention, first, phosphorylated myosin (final concentration, 0.2 mg/ml), actin (final concentration, 0.1 mg/ml), and tropomyosin (final concentration, 30 µg/ml) were added to a buffer that contained 20 mM imidazole-HCl (pH 7.2), 0.1 mM DTT, 100 mM KCl, 2 mM MgC1₂, 1 mM ATP, and 0.1 mM CaC1₂. Then the purified polypeptide was added and the mixture was kept at 30°C for 5 minutes. After this reaction time, trichloroacetic acid was added to the final concentration of 10% to stop the reaction. The reaction mixture was centrifuged, and the amount of free phosphate in the supernatant was assayed by the method of Youngburg (BBRC, 132, 645-651, 1985). The inhibition caused by the polypeptide was found from the difference in the amount of free phosphate related by ATPase in the presence and absence of the polypeptide. Both of these activities were found in type I, type II, 312AA, 122AA, 118AA, and 116AA. The functional unit of human caldesmon polypeptide was found to be 116AA.

### Results of the invention.

As shown by the results described above, this invention provides the complete amino acid sequence of human caldesmon, and the complete DNA sequence that codes for human caldesmon, and it identifies the functional unit of caldesmon activity. These genes and the polypeptides are useful in the fields of biochemistry, medicine, including diagnosis, and the like.

### Explanation of the figures.

Figure 1 is a restriction map of the cDNA that codes for type I and type II caldesmon (CaD). Figure 2 is a figure that shows the relationships of type I, type II, 312AA, 118AA, and 90AA.

SEQ ID NO:1
LENGTH:116 amino acids
TYPE:amino acid
STRANDEDNESS:single
TOPOLOGY:linear
MOLECULE TYPE:peptide
SEQUENCE DESCRIPTION:SEQ ID NO:1:
Arg Leu Glu Gln Tyr Thr Ser Ala Ile Glu Gly Thr Lys Ser Ala
1               5                   10                  15
Lys Pro Thr Lys Pro Ala Ala Ser Asp Leu Pro Val Pro Ala Glu
                20                  25                  30
Gly Val Arg Asn Ile Lys Ser Met Trp Glu Lys Gly Asn Val Phe
                35                  40                  45
Ser Ser Pro Thr Ala Ala Gly Thr Pro Asn Lys Glu Thr Ala Gly
                50                  55                  60
Leu Lys Val Gly Val Ser Ser Arg Ile Asn Glu Trp Leu Thr Lys
                65                  70                  75
Thr Pro Asp Gly Asn Lys Ser Pro Ala Pro Lys Pro Ser Asp Leu
                80                  85                  90
Arg Pro Gly Asp Val Ser Ser Lys Arg Asn Leu Trp Glu Lys Gln
                95                  100                 105
Ser Val Asp Lys Val Thr Ser Pro Thr Lys Val
                110                 115

SEQ ID NO:2

LENGTH:118 amino acids

TYPE:amino acid

STRANDEDNESS:single

TOPOLOGY:linear

MOLECULE TYPE:peptide

SEQUENCE DESCRIPTION:SEQ ID NO:2:

Met Ala Arg Leu Glu Gln Tyr Thr Ser Ala Ile Glu Gly Thr Lys

1               5                   10                  15

Ser Ala Lys Pro Thr Lys Pro Ala Ala Ser Asp Leu Pro Val Pro

                20                  25                  30

Ala Glu Gly Val Arg Asn Ile Lys Ser Met Trp Glu Lys Gly Asn

                35                  40                  45

Val Phe Ser Ser Pro Thr Ala Ala Gly Thr Pro Asn Lys Glu Thr

                50                  55                  60

Ala Gly Leu Lys Val Gly Val Ser Ser Arg Ile Asn Glu Trp Leu

                65                  70                  75

Thr Lys Thr Pro Asp Gly Asn Lys Ser Pro Ala Pro Lys Pro Ser

                80                  85                  90

Asp Leu Arg Pro Gly Asp Val Ser Ser Lys Arg Asn Leu Trp Glu

                95                  100                 105

Lys Gln Ser Val Asp Lys Val Thr Ser Pro Thr Lys Val

                110                 115

SEQ ID NO:3

LENGTH:122 amino acids

TYPE:amino acid

STRANDEDNESS:single

TOPOLOGY:linear

MOLECULE TYPE:peptide

SEQUENCE DESCRIPTION:SEQ ID NO:3:

Met Ala Ile Glu Gly Arg Arg Leu Glu Gln Tyr Thr Ser Ala Ile

1               5                   10                  15

Glu Gly Thr Lys Ser Ala Lys Pro Thr Lys Pro Ala Ala Ser Asp

                20                  25                  30

Leu Pro Val Pro Ala Glu Gly Val Arg Asn Ile Lys Ser Met Trp

                35                  40                  45

Glu Lys Gly Asn Val Phe Ser Ser Pro Thr Ala Ala Gly Thr Pro

                50                  55                  60

Asn Lys Glu Thr Ala Gly Leu Lys Val Gly Val Ser Ser Arg Ile

                65                  70                  75

Asn Glu Trp Leu Thr Lys Thr Pro Asp Gly Asn Lys Ser Pro Ala

                80                  85                  90

Pro Lys Pro Ser Asp Leu Arg Pro Gly Asp Val Ser Ser Lys Arg

                95                  100                 105

Asn Leu Trp Glu Lys Gln Ser Val Asp Lys Val Thr Ser Pro Thr

                110                 115                 120

Lys Val

SEQ ID NO:4

LENGTH:312 amino acids

TYPE:amino acid

STRANDEDNESS:single

TOPOLOGY:linear

MOLECULE TYPE:peptide

SEQUENCE DESCRIPTION:SEQ ID NO:4:

Met Asp Arg Lys Lys Gly Phe Thr Glu Val Lys Ser Gln Asn Gly

1               5                   10                  15

Glu Phe Met Thr His Lys Leu Lys His Thr Glu Asn Thr Phe Ser

                20                  25                  30

Arg Pro Gly Gly Arg Ala Ser Val Asp Thr Lys Glu Ala Glu Gly

                35                  40                  45

Ala Pro Gln Val Glu Ala Gly Lys Arg Leu Glu Glu Leu Arg Arg

                50                  55                  60

Arg Arg Gly Glu Thr Glu Ser Glu Glu Phe Glu Lys Leu Lys Gln

                65                  70                  75

Lys Gln Gln Glu Ala Ala Leu Glu Leu Glu Glu Leu Lys Lys Lys

                80                  85                  90

Arg Glu Glu Arg Arg Lys Val Leu Glu Glu Glu Glu Gln Arg Arg

                95                  100                 105

Lys Gln Glu Glu Ala Asp Arg Lys Leu Arg Glu Glu Glu Glu Lys

                110                 115                 120

Arg Arg Leu Lys Glu Glu Ile Glu Arg Arg Arg Ala Glu Ala Ala

                125                 130                 135

Glu Lys Arg Gln Lys Met Pro Glu Asp Gly Leu Ser Asp Asp Lys

                140                 145                 150

Lys Pro Phe Lys Cys Phe Thr Pro Lys Gly Ser Ser Leu Lys Ile

                155                 160                 165

Glu Glu Arg Ala Glu Phe Leu Asn Lys Ser Val Gln Lys Ser Ser

SEQ ID NO:5
LENGTH:532 amino acids
TYPE:amino acid
STRANDEDNESS:linear
TOPOLOGY:linear
MOLECULE TYPE:peptide
SEQUENCE DESCRIPTION:SEQ ID NO:5:
Met Leu Gly Gly Ser Gly Ser His Gly Arg Arg Ser Leu Ala Ala
1               5                   10                  15
Leu Ser Gln Ile Ala Tyr Gln Arg Asn Asp Asp Asp Glu Glu Glu
                20                  25                  30
Ala Ala Arg Glu Arg Arg Arg Arg Ala Arg Gln Glu Arg Leu Arg
                35                  40                  45
Gln Lys Gln Glu Glu Glu Ser Leu Gly Gln Val Thr Asp Gln Val
                50                  55                  60
Glu Val Asn Ala Gln Asn Ser Val Pro Asp Glu Glu Ala Lys Thr
                65                  70                  75
Thr Thr Thr Asn Thr Gln Val Glu Gly Asp Asp Glu Ala Ala Phe
                80                  85                  90
Leu Glu Arg Leu Ala Arg Arg Glu Glu Arg Arg Gln Lys Arg Leu
                95                  100                 105
Gln Glu Ala Leu Glu Arg Gln Lys Glu Phe Asp Pro Thr Ile Thr
                110                 115                 120
Asp Ala Ser Leu Ser Leu Pro Ser Arg Arg Met Gln Asn Asp Thr
                125                 130                 135
Ala Glu Asn Glu Thr Thr Glu Lys Glu Glu Lys Ser Glu Ser Arg
                140                 145                 150
Gln Glu Arg Tyr Glu Ile Glu Glu Thr Glu Thr Val Thr Lys Ser
                155                 160                 165
Tyr Gln Lys Asn Asp Trp Arg Asp Ala Glu Glu Asn Lys Lys Glu
                170                 175                 180

Asp Lys Glu Lys Glu Glu Glu Glu Glu Glu Lys Pro Lys Arg Gly
                185                 190                 195
Ser Ile Gly Glu Asn Gln Ile Lys Asp Glu Lys Ile Lys Lys Asp
                200                 205                 210
Lys Glu Pro Lys Glu Glu Val Lys Ser Phe Met Asp Arg Lys Lys
                215                 220                 225
Gly Phe Thr Glu Val Lys Ser Gln Asn Gly Glu Phe Met Thr His
                230                 235                 240
Lys Leu Lys His Thr Glu Asn Thr Phe Ser Arg Pro Gly Gly Arg
                245                 250                 255
Ala Ser Val Asp Thr Lys Glu Ala Glu Gly Ala Pro Gln Val Glu
                260                 265                 270
Ala Gly Lys Arg Leu Glu Glu Leu Arg Arg Arg Arg Gly Glu Thr
                275                 280                 285
Glu Ser Glu Glu Phe Glu Lys Leu Lys Gln Lys Gln Gln Glu Ala
                290                 295                 300
Ala Leu Glu Leu Glu Glu Leu Lys Lys Lys Arg Glu Glu Arg Arg
                305                 310                 315
Lys Val Leu Glu Glu Glu Glu Gln Arg Arg Lys Gln Glu Glu Ala
                320                 325                 330
Asp Arg Lys Leu Arg Glu Glu Glu Glu Lys Arg Arg Leu Lys Glu
                335                 340                 345
Glu Ile Glu Arg Arg Arg Ala Glu Ala Ala Glu Lys Arg Gln Lys
                350                 355                 360
Met Pro Glu Asp Gly Leu Ser Asp Asp Lys Lys Pro Phe Lys Cys
                365                 370                 375
Phe Thr Pro Lys Gly Ser Ser Leu Lys Ile Glu Glu Arg Ala Glu
                380                 385                 390
Phe Leu Asn Lys Ser Val Gln Lys Ser Ser Gly Val Lys Ser Thr
                395                 400                 405
His Gln Ala Ala Ile Val Ser Lys Ile Asp Ser Arg Leu Glu Gln

                410                 415                 420

Tyr Thr Ser Ala Ile Glu Gly Thr Lys Ser Ala Lys Pro Thr Lys

                425                 430                 435

Pro Ala Ala Ser Asp Leu Pro Val Pro Ala Glu Gly Val Arg Asn

                440                 445                 450

Ile Lys Ser Met Trp Glu Lys Gly Asn Val Phe Ser Ser Pro Thr

                455                 460                 465

Ala Ala Gly Thr Pro Asn Lys Glu Thr Ala Gly Leu Lys Val Gly

                470                 475                 480

Val Ser Ser Arg Ile Asn Glu Trp Leu Thr Lys Thr Pro Asp Gly

                485                 490                 495

Asn Lys Ser Pro Ala Pro Lys Pro Ser Asp Leu Arg Pro Gly Asp

                500                 505                 510

Val Ser Ser Lys Arg Asn Leu Trp Glu Lys Gln Ser Val Asp Lys

                515                 520                 525

Val Thr Ser Pro Thr Lys Val

                530

SEQ ID NO:6
LENGTH:558 amino acids
TYPE:amino acid
STRANDEDNESS:single
TOPOLOGY:linear
MOLECULE TYPE:peptide
SEQUENCE DESCRIPTION:SEQ ID NO:6:
Met Leu Gly Gly Ser Gly Ser His Gly Arg Arg Ser Leu Ala Ala
1               5                   10                  15
Leu Ser Gln Ile Ala Tyr Gln Arg Asn Asp Asp Asp Glu Glu Glu
                20                  25                  30
Ala Ala Arg Glu Arg Arg Arg Arg Ala Arg Gln Glu Arg Leu Arg
                35                  40                  45
Gln Lys Gln Glu Glu Glu Ser Leu Gly Gln Val Thr Asp Gln Val
                50                  55                  60
Glu Val Asn Ala Gln Asn Ser Val Pro Asp Glu Glu Ala Lys Thr
                65                  70                  75
Thr Thr Thr Asn Thr Gln Val Glu Gly Asp Asp Glu Ala Ala Phe
                80                  85                  90
Leu Glu Arg Leu Ala Arg Arg Glu Glu Arg Arg Gln Lys Arg Leu
                95                  100                 105
Gln Glu Ala Leu Glu Arg Gln Lys Glu Phe Asp Pro Thr Ile Thr
                110                 115                 120
Asp Ala Ser Leu Ser Leu Pro Ser Arg Arg Met Gln Asn Asp Thr
                125                 130                 135
Ala Glu Asn Glu Thr Thr Glu Lys Glu Glu Lys Ser Glu Ser Arg
                140                 145                 150
Gln Glu Arg Tyr Glu Ile Glu Glu Thr Glu Thr Val Thr Lys Ser
                155                 160                 165
Tyr Gln Lys Asn Asp Trp Arg Asp Ala Glu Glu Asn Lys Lys Glu
                170                 175                 180

Asp Lys Glu Lys Glu Glu Glu Glu Glu Glu Lys Pro Lys Arg Gly
                185                 190                 195
Ser Ile Gly Glu Asn Gln Gly Glu Glu Lys Gly Thr Lys Val Gln
                200                 205                 210
Ala Lys Arg Glu Lys Leu Gln Glu Asp Lys Pro Thr Phe Lys Lys
                215                 220                 225
Glu Glu Ile Lys Asp Glu Lys Ile Lys Lys Asp Lys Glu Pro Lys
                230                 235                 240
Glu Glu Val Lys Ser Phe Met Asp Arg Lys Lys Gly Phe Thr Glu
                245                 250                 255
Val Lys Ser Gln Asn Gly Glu Phe Met Thr His Lys Leu Lys His
                260                 265                 270
Thr Glu Asn Thr Phe Ser Arg Pro Gly Gly Arg Ala Ser Val Asp
                275                 280                 285
Thr Lys Glu Ala Glu Gly Ala Pro Gln Val Glu Ala Gly Lys Arg
                290                 295                 300
Leu Glu Glu Leu Arg Arg Arg Arg Gly Glu Thr Glu Ser Glu Glu
                305                 310                 315
Phe Glu Lys Leu Lys Gln Lys Gln Gln Glu Ala Ala Leu Glu Leu
                320                 325                 330
Glu Glu Leu Lys Lys Lys Arg Glu Glu Arg Arg Lys Val Leu Glu
                335                 340                 345
Glu Glu Glu Gln Arg Arg Lys Gln Glu Glu Ala Asp Arg Lys Leu
                350                 355                 360
Arg Glu Glu Glu Glu Lys Arg Arg Leu Lys Glu Glu Ile Glu Arg
                365                 370                 375
Arg Arg Ala Glu Ala Ala Glu Lys Arg Gln Lys Met Pro Glu Asp
                380                 385                 390
Gly Leu Ser Asp Asp Lys Lys Pro Phe Lys Cys Phe Thr Pro Lys
                395                 400                 405
Gly Ser Ser Leu Lys Ile Glu Glu Arg Ala Glu Phe Leu Asn Lys

                410                 415                 420
Ser Val Gln Lys Ser Ser Gly Val Lys Ser Thr His Gln Ala Ala
                425                 430                 435
Ile Val Ser Lys Ile Asp Ser Arg Leu Glu Gln Tyr Thr Ser Ala
                440                 445                 450
Ile Glu Gly Thr Lys Ser Ala Lys Pro Thr Lys Pro Ala Ala Ser
                455                 460                 465
Asp Leu Pro Val Pro Ala Glu Gly Val Arg Asn Ile Lys Ser Met
                470                 475                 480
Trp Glu Lys Gly Asn Val Phe Ser Ser Pro Thr Ala Ala Gly Thr
                485                 490                 495
Pro Asn Lys Glu Thr Ala Gly Leu Lys Val Gly Val Ser Ser Arg
                500                 505                 510
Ile Asn Glu Trp Leu Thr Lys Thr Pro Asp Gly Asn Lys Ser Pro
                515                 520                 525
Ala Pro Lys Pro Ser Asp Leu Arg Pro Gly Asp Val Ser Ser Lys
                530                 535                 540
Arg Asn Leu Trp Glu Lys Gln Ser Val Asp Lys Val Thr Ser Pro
                545                 550                 555
Thr Lys Val

SEQ ID NO:7

LENGTH:348 base pairs

TYPE:nucleic acid

STRANDEDNESS:double

TOPOLOGY:linear

MOLECULE TYPE: cDNA to mRNA

ORIGINAL SOURCE:Human

IMMEDIATE SOURCE:Hela cell

SEQUENCE DESCRIPTION:SEQ ID NO:7:

AGACTGGAGC AGTATACCAG TGCAATTGAG GGAACAAAAA GCGCAAAACC TACAAAGCCG 60

GCAGCCTCGG ATCTTCCTGT TCCTGCTGAA GGTGTACGCA ACATCAAGAG TATGTGGGAG 120

AAAGGAAATG TGTTTTCATC CCCCACTGCA GCAGGCACAC CAAATAAGGA AACTGCTGGC 180

TTGAAGGTAG GGGTTTCTAG CCGTATCAAT GAATGGCTAA CTAAAACCCC AGATGGAAAC 240

AAGTCACCTG CTCCCAAACC TTCTGACTTG AGACCAGGAG ACGTATCCAG CAAGCGGAAC 300

CTCTGGGAAA AGCAATCTGT GGATAAGGTC ACTTCCCCCA CTAAGGTT 348

SEQ ID NO:8

LENGTH:357 base pairs

TYPE:nucleic acid

STRANDEDNESS:double

TOPOLOGY:linear

MOLECULE TYPE: cDNA to mRNA

ORIGINAL SOURCE:Human

IMMEDIATE SOURCE:Hela cell

SEQUENCE DESCRIPTION:SEQ ID NO:8:

ATGGCCAGAC TGGAGCAGTA TACCAGTGCA ATTGAGGGAA CAAAAAGCGC AAAACCTACA 60

AAGCCGGCAG CCTCGGATCT TCCTGTTCCT GCTGAAGGTG TACGCAACAT CAAGAGTATG 120

TGGGAGAAAG GGAATGTGTT TTCATCCCCC ACTGCAGCAG GCACACCAAA TAAGGAAACT 180

GCTGGCTTGA AGGTAGGGGT TTCTAGCCGT ATCAATGAAT GGCTAACTAA AACCCCAGAT 240

GGAAACAAGT CACCTGCTCC CAAACCTTCT GACTTGAGAC CAGGAGACGT ATCCAGCAAG 300

CGGAACCTCT GGGAAAAGCA ATCTGTGGAT AAGGTCACTT CCCCCACTAA GGTTTGA 357

SEQ ID NO:9

LENGTH:369 base pairs

TYPE:nucleic acid

STRANDEDNESS:double

TOPOLOGY:linear

MOLECULE TYPE: cDNA to mRNA

ORIGINAL SOURCE:Human

IMMEDIATE SOURCE:Hela cell

SEQUENCE DESCRIPTION:SEQ ID NO:9:

ATGGCCATCG AGGGTAGGAG ACTGGAGCAG TATACCAGTG CAATTGAGGG AACAAAAAGC 60

GCAAAACCTA CAAAGCCGGC AGCCTCGGAT CTTCCTGTTC CTGCTGAAGG TGTACGCAAC 120

ATCAAGAGTA TGTGGGAGAA AGGGAATGTG TTTTCATCCC CCACTGCAGC AGGCACACCA 180

AATAAGGAAA CTGCTGGCTT GAAGGTAGGG GTTTCTAGCC GTATCAATGA ATGGCTAACT 240

AAAACCCCAG ATGGAAACAA GTCACCTGCT CCCAAACCTT CTGACTTGAG ACCAGGAGAC 300

GTATCCAGCA AGCGGAACCT CTGGGAAAAG CAATCTGTGG ATAAGGTCAC TTCCCCCACT 360

AAGGTTTGA 369

SEQ ID NO:10

LENGTH:939 base pairs

TYPE:nucleic acid

STRANDEDNESS:double

TOPOLOGY:linear

MOLECULE TYPE: cDNA to mRNA

ORIGINAL SOURCE:Human

IMMEDIATE SOURCE:Hela cell

SEQUENCE DESCRIPTION:SEQ ID NO:10:

ATGGATCGAA AGAAGGGATT TACAGAAGTT AAGTCGCAGA ATGGAGAATT CATGACCCAC 60

AAACTTAAAC ATACTGAGAA TACTTTCAGC CGCCCTGGAG GGAGGGCCAG CGTGGACACC 120

AAGGAGGCTG AGGGCGCCCC CCAGGTGGAA GCCGGCAAAA GGCTGGAGGA GCTTCGTCGT 180

CGTCGCGGGG AGACCGAGAG CGAAGAGTTC GAGAAGCTCA AACAGAAGCA GCAGGAGGCG 240

GCTTTGGAGC TGGAGGAACT CAAGAAAAAG AGGGAGGAGA GAAGGAAGGT CCTGGAGGAG 300

GAAGAGCAGA GGAGGAAGCA GGAGGAAGCC GATCGAAAAC TCAGAGAGGA GGAAGAGAAG 360

AGGAGGCTAA AGGAAGAGAT TGAAAGGCGA AGAGCAGAAG CTGCTGAGAA ACGCCAGAAG 420

ATGCCAGAAG ATGGCTTGTC AGATGACAAG AAACCATTCA AGTGTTTCAC TCCTAAAGGT 480

TCATCTCTCA AGATAGAAGA GCGAGCAGAA TTTTTGAATA AGTCTGTGCA GAA[?]AGCAGT 540

GGTGTCAAAT CGACCCATCA AGCAGCAATA GTCTCCAAGA TTGACAGCAG ACTGGAGCAG 600

TATACCAGTG CAATTGAGGG AACAAAAAGC GCAAAACCTA CAAAGCCGGC AGCCTCGGAT 660

CTTCCTGTTC CTGCTGAAGG TGTACGCAAC ATCAAGAGTA TGTGGGAGAA AGGGAATGTG 720

TTTTCATCCC CCACTGCAGC AGGCACACCA AATAAGGAAA CTGCTGGCTT GAAGGTAGGG 780

GTTTCTAGCC GTATCAATGA ATGGCTAACT AAAACCCCAG ATGGAAACAA GTCACCTGCT 840

CCCAAACCTT CTGACTTGAG ACCAGGAGAC GTATCCAGCA AGCGGAACCT CTGGGAAAAG 900

CAATCTGTGG ATAAGGTCAC TTCCCCCACT AAGGTTTGA 939

SEQ ID NO:11

LENGTH:1599 base pairs

TYPE:nucleic acld

STRANDEDNESS:double

TOPOLOGY:linear

MOLECULE TYPE: cDNA to mRNA

ORIGINAL SOURCE:Human

IMMEDIATE SOURCE : Hela cell

SEQUENCE DESCRIPTION:SEQ ID NO:11:

ATGCTGGGTG GATCCGGATC GCATGGAAGA CGCAGCCTGG CCGCGCTCTC CCAAATCGCC 60

TACCAGAGGA ATGACGATGA TGAAGAGGAG GCAGCCCGGG AACGGCGCCG CCGAGCCCGA 120

CAGGAACGGC TGCGGCAGAA GCAGGAGGAA GAATCCTTGG GACAGGTGAC CGACCAGGTG 180

GAGGTGAATG CCCAGAACAG TGTGCCTGAC GAGGAGGCCA AGACAACCAC CACAAACACT 240

CAAGTGGAAG GGGATGATGA GGCCGCATTC CTGGAGCGCC TGGCTCGGCG TGAGGAAAGA 300

CGCCAAAAAC GCCTTCAGGA GGCTCTGGAG CGGCAGAAGG AGTTCGACCC AACAATAACA 360

GATGCAAGTC TGTCGCTCCC AAGCAGAAGA ATGCAAAATG ACACAGCAGA AAATGAAACT 420

ACCGAGAAGG AAGAAAAAAG TGAAAGTCGC CAAGAAAGAT ACGAGATAGA GGAAACAGAA 480

ACAGTCACCA AGTCCTACCA GAAGAATGAT TGGAGGGATG CTGAAGAAAA CAAGAAAGAA 540

GACAAGGAAA AGGAGGAGGA GGAAGAGGAG AAGCCAAAGC GAGGGAGCAT TGGAGAAAAT 600

CAGATCAAAG ATGAAAAGAT TAAAAAGGAC AAAGAACCCA AAGAAGAAGT TAAGAGCTTC 660

ATGGATCGAA AGAAGGGATT TACAGAAGTT AAGTCGCAGA ATGGAGAATT CATGACCCAC 720

AAACTTAAAC ATACTGAGAA TACTTTCAGC CGCCCTGGAG GGAGGGCCAG CGTGGACACC 780

AAGGAGGCTG AGGGCGCCCC CCAGGTGGAA GCCGGCAAAA GGCTGGAGGA GCTTCGTCGT 840

CGTCGCGGGG AGACCGAGAG CGAAGAGTTC GAGAAGCTCA AACAGAAGCA GCAGGAGGCG 900

GCTTTGGAGC TGGAGGAACT CAAGAAAAAG AGGGAGGAGA GAAGGAAGGT CCTGGAGGAG 960

GAAGAGCAGA GGAGGAAGCA GGAGGAAGCC GATCGAAAAC TCAGAGAGGA GGAAGAGAAG 1020

AGGAGGCTAA AGGAAGAGAT TGAAAGGCGA AGAGCAGAAG CTGCTGAGAA ACGCCAGAAG 1080

ATGCCAGAAG ATGGCTTGTC AGATGACAAG AAACCATTCA AGTGTTTCAC TCCTAAAGGT 1140

TCATCTCTCA AGATAGAAGA GCGAGCAGAA TTTTTGAATA AGTCTGTGCA GAAAAGCAGT 1200

GGTGTCAAAT CGACCCATCA AGCAGCAATA GTCTCCAAGA TTGACAGCAG ACTGGAGCAG 1260

TATACCAGTG CAATTGAGGG AACAAAAAGC GCAAAACCTA CAAAGCCGGC AGCCTCGGAT 1320

CTTCCTGTTC CTGCTGAAGG TGTACGCAAC ATCAAGAGTA TGTGGAGAAA AGGGAATGTG 1380

TTTTCATCCC CCACTGCAGC AGGCACACCA AATAAGGAAA CTGCTGGCTT GAAGGTAGGG 1440

GTTTCTAGCC GTATCAATGA ATGGCTAACT AAAACCCCAG ATGGAAACAA GTCACCTGCT 1500

[?]CCAAACCTT CTGACTTGAG ACCAGGAGAC GTATCCAGCA AGCGGAACCT CTGGGAAAAG 1560

CAATCTGTGG ATAAGGTCAC TTCCCCCACT AAGGTTTGA 1599

SEQ ID NO:12

LENGTH:1677 base pairs

TYPE:nucleic acid

STRANDEDNESS:double

TOPOLOGY:linear

MOLECULE TYPE: cDNA to mRNA

ORIGINAL SOURCE:Human

IMMEDIATE SOURCE:Hela cell

SEQUENCE DESCRIPTION:SEQ ID NO:12:

ATGCTGGGTG GATCCGGATC GCATGGAAGA CGCAGCCTGG CCGCGCTCTC CCAAATCGCC 60

TACCAGAGGA ATGACGATGA TGAAGAGGAG GCAGCCCGGG AACGGCGCCG CCGAGCCCGA 120

CAGGAACGGC TGCGGCAGAA GCAGGAGGAA GAATCCTTGG GACAGGTGAC CGACCAGGTG 180

GAGGTGAATG CCCAGAACAG TGTGCCTGAC GAGGAGGCCA AGACAACCAC CACAAACACT 240

CAAGTGGAAG GGGATGATGA GGCCGCATTC CTGGAGCGCC TGGCTCGGCG TGAGGAAAGA 300

CGCCAAAAAC GCCTTCAGGA GGCTCTGGAG CGGCAGAAGG AGTTCGACCC AACAATAACA 360

GATGCAAGTC TGTCGCTCCC AAGCAGAAGA ATGCAAAATG ACACAGCAGA AAATGAAACT 420

ACCGAGAAGG AAGAAAAAAG TGAAAGTCGC CAAGAAAGAT ACGAGATAGA GGAAACAGAA 480

ACAGTCACCA AGTCCTACCA GAAGAATGAT TGGAGGGATG CTGAAGAAAA CAAGAAAGAA 540

GACAAGGAAA AGGAGGAGGA GGAAGAGGAG AAGCCAAAGC GAGGGAGCAT TGGAGAAAAT 600

CAGGGAGAAG AGAAGGGAAC TAAAGTGCAA GCTAAAAGAG AAAAGCTCCA AGAAGACAAG 660

[?]CTACCTTCA AAAAAGAAGA GATCAAAGAT GAAAAGATTA AAAAGGACAA AGAACCCAAA 720

GAAGAAGTTA AGAGCTTCAT GGATCGAAAG AAGGGATTTA CAGAAGTTAA GTCGCAGAAT 780

GGAGAATTCA TGACCCACAA ACTTAAACAT ACTGAGAATA CTTTCAGCCG CCCTGGAGGG 840

AGGGCCAGCG TGGACACCAA GGAGGCTGAG GGCGCCCCCC AGGTGGAAGC CGGCAAAAGG 900

CTGGAGGAGC TTCGTCGTCG TCGCGGGGAG ACCGAGAGCG AAGAGTTCGA GAAGCTCAAA 960

CAGAAGCAGC AGGAGGCGGC TTTGGAGCTG GAGGAACTCA AGAAAAAGAG GGAGGAGAGA 1020

AGGAAGGTCC TGGAGGAGGA AGAGCAGAGG AGGAAGCAGG AGGAAGCCGA TCGAAAACTC 1080

AGAGAGGAGG AAGAGAAGAG GAGGCTAAAG GAAGAGATTG AAAGGCGAAG AGCAGAAGCT 1140

GCTGAGAAAC GCCAGAAGAT GCCAGAAGAT GGCTTGTCAG ATGACAAGAA ACCATTCAAG 1200

TGTTTCACTC CTAAAGGTTC ATCTCTCAAG ATAGAAGAGC GAGCAGAATT TTTGAATAAG 1260

TCTGTGCAGA AAAGCAGTGG TGTCAAATCG ACCCATCAAG CAGCAATAGT CTCCAAGATT 1320

GACAGCAGAC TGGAGCAGTA TACCAGTGCA ATTGAGGGAA CAAAAAGCGC AAAACCTACA 1380

AAGCCGGCAG CCTCGGATCT TCCTGTTCCT GCTGAAGGTG TACGCAACAT CAAGAGTATG 1440

TGGGAGAAAG GGAATGTGTT TTCATCCCCC ACTGCAGCAG GCACACCAAA TAAGGAAACT 1500

GCTGGCTTGA AGGTAGGGGT TTCTAGCCGT ATCAATGAAT GGCTAACTAA AACCCCAGAT 1560

GGAAACAAGT CACCTGCTCC CAAACCTTCT GACTTGAGAC CAGGAGACGT ATCCAGCAAG 1620

CGGAACCTCT GGGAAAAGCA ATCTGTGGAT AAGGTCACTT CCCCCACTAA GGTTTGA 1677

SEQ ID NO:13
LENGTH:2137 base pairs
[?][?]PE:nucleic acid
STRANDEDNESS:double
TOPOLOGY:linear
MOLECULE TYPE:cDNA to mRNA
ORIGINAL SOURCE:Human
IMMEDIATE SOURCE:Hela cell
FEATURE:
12-1607 CDS ; human caldesmon of lower molecular weight
2025-2030 poly A signal
[?]121-2137 poly A site
SEQUENCE DESCRIPTION:SEQ ID NO:13:
CAACTGCGGA CATGCTGGGT GGATCCGGAT CGCATGGAAG ACGCAGCCTG GCCGCGCTCT 60
CCCAAATCGC CTACCAGAGG AATGACGATG ATGAAGAGGA GGCAGCCCGG GAACGGCGCC 120
GCCGAGCCCG ACAGGAACGG CTGCGGCAGA AGCAGGAGGA AGAATCCTTG GGACAGGTGA 180
CCGACCAGGT GGAGGTGAAT GCCCAGAACA GTGTGCCTGA CGAGGAGGCC AAGACAACCA 240
CCACAAACAC TCAAGTGGAA GGGGATGATG AGGCCGCATT CCTGGAGCGC CTGGCTCGGC 300
GTGAGGAAAG ACGCCAAAAA CGCCTTCAGG AGGCTCTGGA GCGGCAGAAG GAGTTCGACC 360
CAACAATAAC AGATGCAAGT CTGTCGCTCC CAAGCAGAAG AATGCAAAAT GACACAGCAG 420
AAAATGAAAC TACCGAGAAG GAAGAAAAAA GTGAAAGTCG CCAAGAAAGA TACGAGATAG 480
AGGAAACAGA AACAGTCACC AAGTCCTACC AGAAGAATGA TTGGAGGGAT GCTGAAGAAA 540
ACAAGAAAGA AGACAAGGAA AAGGAGGAGG AGGAAGAGGA GAAGCCAAAG CGAGGGAGCA 600
TTGGAGAAAA TCAGATCAAA GATGAAAAGA TTAAAAAGGA CAAAGAACCC AAAGAAGAAG 660
TTAAGAGCTT CATGGATCGA AAGAAGGGAT TTACAGAAGT TAAGTCGCAG AATGGAGAAT 720
TCATGACCCA CAAACTTAAA CATACTGAGA ATACTTTCAG CCGCCCTGGA GGGAGGGCCA 780
GCGTGGACAC CAAGGAGGCT GAGGGCGCCC CCCAGGTGGA AGCCGGCAAA AGGCTGGAGG 840
AGCTTCGTCG TCGTCGCGGG GAGACCGAGA GCGAAGAGTT CGAGAAGCTC AAACAGAAGC 900
AGCAGGAGGC GGCTTTGGAG CTGGAGGAAC TCAAGAAAAA GAGGGAGGAG AGAAGGAAGG 960
TCCTGGAGGA GGAAGAGCAG AGGAGGAAGC AGGAGGAAGC CGATCGAAAA CTCAGAGAGG 1020
AGGAAGAGAA GAGGAGGCTA AAGGAAGAGA TTGAAAGGCG AAGAGCAGAA GCTGCTGAGA 1080

AACGCCAGAA GATGCCAGAA GATGGCTTGT CAGATGACAA GAAACCATTC AAGTGTTTCA 1140

CTCCTAAAGG TTCATCTCTC AAGATAGAAG AGCGAGCAGA ATTTTTGAAT AAGTCTGTGC 1200

AGAAAAGCAG TGGTGTCAAA TCGACCCATC AAGCAGCAAT AGTCTCCAAG ATTGACAGCA 1260

GACTGGAGCA GTATACCAGT GCAATTGAGG GAACAAAAAG CGCAAAACCT ACAAAGCCGG 1320

CAGCCTCGGA TCTTCCTGTT CCTGCTGAAG GTGTACGCAA CATCAAGAGT ATGTGGGAGA 1380

AAGGGAATGT GTTTTCATCC CCCACTGCAG CAGGCACACC AAATAAGGAA ACTGCTGGCT 1440

TGAAGGTAGG GGTTTCTAGC CGTATCAATG AATGGCTAAC TAAAACCCCA GATGGAAACA 1500

AGTCACCTGC TCCCAAACCT TCTGACTTGA GACCAGGAGA CGTATCCAGC AAGCGGAACC 1560

TCTGGGAAAA GCAATCTGTG GATAAGGTCA CTTCCCCCAC TAAGGTTTGA GACAGTTCCA 1620

GAAAGAACCC AAGCTCAAGA CGCAGGACGA GCTCAGTTGT AGAGGGCTAA TTCGCTCTTT 1680

TGTATTTATG TTGATTTACT AAATTGGGTT CATTATCTTT TATTTTTCAA TATCCCAGTA 1740

[?][?]CCCATGTA TATTATCACT ATATTTAATA ATCACAGCTA GAGATGTTCA TGGTAAAAGT 1800

ACTGCCTTTG CACAGGAGCC TGTTTCTAAA GAAACCCATG CTGTGAAATA GAGACTTTTC 1860

TACTGATCAT CATAACTCTG TATCTGAGCA GTGATACCAA CCACATCTGA AGTCAACAGA 1920

AGATCCAAGT TTAAAATTGC CTGCGGAATG TGTGCAGTAT CTAGAAAAAT GAACCGTAGT 1980

TTTGTTTTTT TAAATACAGA AGTCATGTTG TTTCTGCACT TTATAATAAA GCATGGAAGA 2040

AATTATCTTA GTAGGCAATT GTAACACTTT TTGAAAGTAA CCCATTTCAG ATTTGAAATA 2100

CTGCAATAAT GGTTGTCTTT AAAAAAAAAA AAAAAAA 2137

SEQ ID NO:14
LENGTH:2215 base pairs
TYPE:nucleic acid
 TRANDEDNESS:double
TOPOLOGY:linear
MOLECULE TYPE: cDNA to mRNA
ORIGINAL SOURCE:Human
IMMEDIATE SOURCE:Hela cell
FEATURE
 12-1685  CDS; human caldesmon of higher molecular weight
2103-2108  poly A signal
[?]099-2215  poly A site
SEQUENCE DESCRIPTION:SEQ ID NO:14:
CAACTGCGGA CATGCTGGGT GGATCCGGAT CGCATGGAAG ACGCAGCCTG GCCGCGCTCT   60
CCCAAATCGC CTACCAGAGG AATGACGATG ATGAAGAGGA GGCAGCCCGG GAACGGCGCC  120
GCCGAGCCCG ACAGGAACGG CTGCGGCAGA AGCAGGAGGA AGAATCCTTG GGACAGGTGA  180
CCGACCAGGT GGAGGTGAAT GCCCAGAACA GTGTGCCTGA CGAGGAGGCC AAGACAACCA  240
CCACAAACAC TCAAGTGGAA GGGGATGATG AGGCCGCATT CCTGGAGCGC CTGGCTCGGC  300
GTGAGGAAAG ACGCCAAAAA CGCCTTCAGG AGGCTCTGGA GCGGCAGAAG GAGTTCGACC  360
CAACAATAAC AGATGCAAGT CTGTCGCTCC CAAGCAGAAG AATGCAAAAT GACACAGCAG  420
[?][?]AATGAAAC TACCGAGAAG GAAGAAAAAA GTGAAAGTCG CCAAGAAAGA TACGAGATAG  480
AGGAAACAGA AACAGTCACC AAGTCCTACC AGAAGAATGA TTGGAGGGAT GCTGAAGAAA  540
ACAAGAAAGA AGACAAGGAA AAGGAGGAGG AGGAAGAGGA GAAGCCAAAG CGAGGGAGCA  600
TTGGAGAAAA TCAGGGAGAA GAGAAGGGAA CTAAAGTGCA AGCTAAAAGA GAAAAGCTCC  660
AAGAAGACAA GCCTACCTTC AAAAAAGAAG AGATCAAAGA TGAAAAGATT AAAAAGGACA  720
AAGAACCCAA AGAAGAAGTT AAGAGCTTCA TGGATCGAAA GAAGGGATTT ACAGAAGTTA  780
AGTCGCAGAA TGGAGAATTC ATGACCCACA AACTTAAACA TACTGAGAAT ACTTTCAGCC  840
GCCCTGGAGG GAGGGCCAGC GTGGACACCA AGGAGGCTGA GGGCGCCCCC CAGGTGGAAG  900
CCGGCAAAAG GCTGGAGGAG CTTCGTCGTC GTCGCGGGGA GACCGAGAGC GAAGAGTTCG  960
AGAAGCTCAA ACAGAAGCAG CAGGAGGCGG CTTTGGAGCT GGAGGAACTC AAGAAAAAGA 1020
GGGAGGAGAG AAGGAAGGTC CTGGAGGAGG AAGAGCAGAG GAGGAAGCAG GAGGAAGCCG 1080

ATCGAAAACT CAGAGAGGAG GAAGAGAAGA GGAGGCTAAA GGAAGAGATT GAAAGGCGAA 1140
GAGCAGAAGC TGCTGAGAAA CGCCAGAAGA TGCCAGAAGA TGGCTTGTCA GATGACAAGA 1200
AACCATTCAA GTGTTTCACT CCTAAAGGTT CATCTCTCAA GATAGAAGAG CGAGCAGAAT 1260
TTGAATAA GTCTGTGCAG AAAAGCAGTG GTGTCAAATC GACCCATCAA GCAGCAATAG 1320
TCTCCAAGAT TGACAGCAGA CTGGAGCAGT ATACCAGTGC AATTGAGGGA ACAAAAAGCG 1380
CAAAACCTAC AAAGCCGGCA GCCTCGGATC TTCCTGTTCC TGCTGAAGGT GTACGCAACA 1440
TCAAGAGTAT GTGGGAGAAA GGGAATGTGT TTTCATCCCC CACTGCAGCA GGCACACCAA 1500
ATAAGGAAAC TGCTGGCTTG AAGGTAGGGG TTTCTAGCCG TATCAATGAA TGGCTAACTA 1560
AAACCCCAGA TGGAAACAAG TCACCTGCTC CCAAACCTTC TGACTTGAGA CCAGGAGACG 1620
TATCCAGCAA GCGGAACCTC TGGGAAAAGC AATCTGTGGA TAAGGTCACT TCCCCCACTA 1680
AGGTTTGAGA CAGTTCCAGA AAGAACCCAA GCTCAAGACG CAGGACGAGC TCAGTTGTAG 1740
[?]GGGCTAATT CGCTCTTTTG TATTTATGTT GATTTACTAA ATTGGGTTCA TTATCTTTTA 1800
TTTTTCAATA TCCCAGTAAA CCCATGTATA TTATCACTAT ATTTAATAAT CACAGCTAGA 1860
GATGTTCATG GTAAAAGTAC TGCCTTTGCA CAGGAGCCTG TTTCTAAAGA AACCCATGCT 1920
GTGAAATAGA GACTTTTCTA CTGATCATCA TAACTCTGTA TCTGAGCAGT GATACCAACC 1980
ACATCTGAAG TCAACAGAAG ATCCAAGTTT AAAATTGCCT GCGGAATGTG TGCAGTATCT 2040
AGAAAAATGA ACCGTAGTTT TGTTTTTTTA AATACAGAAG TCATGTTGTT TCTGCACTTT 2100
ATAATAAAGC ATGGAAGAAA TTATCTTAGT AGGCAATTGT AACACTTTTT GAAAGTAACC 2160
CATTTCAGAT TTGAAATACT GCAATAATGG TTGTCTTTAA AAAAAAAAAA AAAAA 2215

SEQ ID NO:15

LENGTH:29 bases

TYPE:nucleic acid

STRANDEDNESS:single

TOPOLOGY:linear

MOLECULE TYPE:Other nucleic acid (synthetic DNA)

ANTI-SENSE:NO

SEQUENCE DESCRIPTION:SEQ ID NO:15

CAGACCATGG ATCGAAAGAA GGGATTTAC 29

SEQ ID NO:16

LENGTH:30 bases

TYPE:nucleic acid

STRANDEDNESS:single

TOPOLOGY:linear

MOLECULE TYPE:Other nucleic acid (synthetic DNA)

ANTI-SENSE:YES

SEQUENCE DESCRIPTION:SEQ ID NO:16:

TACCGAGCTC AAACCTTAGT GGGGGAAGTG 30

SEQ ID NO:17
LENGTH:47 bases
TYPE:nucleic acid
STRANDEDNESS:single
TOPOLOGY:linear
MOLECULE TYPE:Other nucleic acid (synthetic DNA)
ANTI-SENSE:NO
SEQUENCE DESCRIPTION:SEQ ID NO:17:
CAGACCATGG CCATCGAGGG TAGGAGACTG GAGCAGTATA CCAGTGC 47

SEQ ID NO:18
LENGTH:30 bases
TYPE:nucleic acid
STRANDEDNESS:single
TOPOLOGY:linear
MOLECULE TYPE:Other nucleic acid (synthetic DNA)
ANTI-SENSE:YES
SEQUENCE DESCRIPTION:SEQ ID NO:18:
GCTCGAATTC AAACCTTAGT GGGGGAAGTG 30

SEQ ID NO:19

LENGTH:30 bases

TYPE:nucleic acid

STRANDEDNESS:single

TOPOLOGY:linear

MOLECULE TYPE:Other nucleic acid (synthetic DNA)

ANTI-SENSE:NO

SEQUENCE DESCRIPTION:SEQ ID NO:19:

CAGACCATGG CCAGACTGGA GCAGTATACC 30

SEQ ID NO:20

LENGTH:42 bases

TYPE:nucleic acid

STRANDEDNESS:single

TOPOLOGY:linear

MOLECULE TYPE:Other nucleic acid (synthetic DNA)

ANTI-SENSE:NO

SEQUENCE DESCRIPTION:SEQ ID NO:20:

CAGACCATGG CCATCGAGGG TAGGATTGAG GGAACAAAAA GC 42

SEQ ID NO:21
LENGTH:26 bases
TYPE:nucleic acid
STRANDEDNESS:single
TOPOLOGY:linear
MOLECULE TYPE:Other nucleic acid (synthetic DNA)
ANTI-SENSE:YES
SEQUENCE DESCRIPTION:SEQ ID NO:21:
GCTCGAATTC AGGATACGTC TCCTGG 26

SEQ ID NO:23

LENGTH:285 base pairs

TYPE:nucleic acid

STRANDEDNESS:double

TOPOLOGY:linear

MOLECULE TYPE:cDNA to mRNA

ORIGINAL SOURCE:Human

IMMEDIATE SOURCE:Hela cell

SEQUENCE DESCRIPTION:SEQ ID NO:23:

ATGGCCATCG AGGGTAGGAT TGAGGGAACA AAAAGCGCAA AACCTACAAA GCCGGCAGCC 60

TCGGATCTTC CTGTTCCTGC TGAAGGTGTA CGCAACATCA AGAGTATGTG GGAGAAAGGG 120

AATGTGTTTT CATCCCCCAC TGCAGCAGGC ACACCAAATA AGGAAACTGC TGGCTTGAAG 180

GTAGGGGTTT CTAGCCGTAT CAATGAATGG CTAACTAAAA CCCCAGATGG AAACAAGTCA 240

CCTGCTCCCA AACCTTCTGA CTTGAGACCA GGAGACGTAT CCTGA 285

SEQ ID NO:24

LENGTH:30 bases

TYPE:nucleic acid

STRANDEDNESS:single

TOPOLOGY:linear

MOLECULE TYPE:Other nucleic acid (synthetic DNA)

ANTI-SENSE:NO

SEQUENCE DESCRIPTION:SEQ ID NO:24:

CAGACCATGG CCATTGAGGG AACAAAAAGC 30

SEQ ID NO:25
LENGTH:90 amino acids
TYPE:amino acid
STRANDEDNESS:single
TOPOLOGY:linear
MOLECULE TYPE:peptide
SEQUENCE DESCRIPTION:SEQ ID NO:25:
Met Ala Ile Glu Gly Thr Lys Ser Ala Lys Pro Thr Lys Pro Ala
1               5                   10                  15
Ala Ser Asp Leu Pro Val Pro Ala Glu Gly Val Arg Asn Ile Lys
                20                  25                  30
Ser Met Trp Glu Lys Gly Asn Val Phe Ser Ser Pro Thr Ala Ala
                35                  40                  45
Gly Thr Pro Asn Lys Glu Thr Ala Gly Leu Lys Val Gly Val Ser
                50                  55                  60
Ser Arg Ile Asn Glu Trp Leu Thr Lys Thr Pro Asp Gly Asn Lys
                65                  70                  75
Ser Pro Ala Pro Lys Pro Ser Asp Leu Arg Pro Gly Asp Val Ser
                80                  85                  90

SEQ ID NO:26

LENGTH:273 base pairs

TYPE:nucleic acid

STRANDEDNESS:double

TOPOLOGY:linear

MOLECULE TYPE:cDNA to mRNA

ORIGINAL SOURCE:Human

IMMEDIATE SOURCE:Hela cell

SEQUENCE DESCRIPTION:SEQ ID NO:26:

ATGGCCATTG AGGGAACAAA AAGCGCAAAA CCTACAAAGC CGGCAGCCTC GGATCTTCCT 60

GTTCCTGCTG AAGGTGTACG CAACATCAAG AGTATGTGGG AGAAAAGGAA TGTGTTTTCA 120

TCCCCCACTG CAGCAGGCAC ACCAAATAAG GAAACTGCTG GCTTGAAGGT AGGGGTTTCT 180

AGCCGTATCA ATGAATGGCT AACTAAAACC CCAGATGGAA ACAAGTCACC TGCTCCCAAA 240

CCTTCTGACT TGAGACCAGG AGACGTATCC TGA 273

SEQ ID NO:27
LENGTH:88 amino acids
TYPE:amino acid
STRANDEDNESS:single
TOPOLOGY:linear
MOLECULE TYPE:peptide
SEQUENCE DESCRIPTION:SEQ ID NO:27:
Ile Glu Gly Thr Lys Ser Ala Lys Pro Thr Lys Pro Ala Ala Ser
1               5                   10                  15
Asp Leu Pro Val Pro Ala Glu Gly Val Arg Asn Ile Lys Ser Met
                20                  25                  30
Trp Glu Lys Gly Asn Val Phe Ser Ser Pro Thr Ala Ala Gly Thr
                35                  40                  45
Pro Asn Lys Glu Thr Ala Gly Leu Lys Val Gly Val Ser Ser Arg
                50                  55                  60
Ile Asn Glu Trp Leu Thr Lys Thr Pro Asp Gly Asn Lys Ser Pro
                65                  70                  75
Ala Pro Lys Pro Ser Asp Leu Arg Pro Gly Asp Val Ser
                80                  85

SEQ ID NO:28
LENGTH:6 amino acids
TYPE:amino acid
STRANDEDNESS:single
TOPOLOGY:linear
MOLECULE TYPE:peptide
SEQUENCE DESCRIPTION:SEQ ID NO:28:
Met Ala Ile Glu Gly Arg
 1               5

SEQ ID NO:29

LENGTH:20 bases

TYPE:nucleic acid

STRANDEDNESS:single

TOPOLOGY:linear

MOLECULE TYPE:Other nucleic acid (synthetic DNA)

ANTI-SENSE:YES

SEQUENCE DESCRIPTION:SEQ ID NO:29:

CTACAACTGA GCTCGTCCTG 20

SEQ ID NO:30

LENGTH:22 bases

TYPE:nucleic acid

STRANDEDNESS:single

TOPOLOGY:linear

MOLECULE TYPE:Other nucleic acid (synthetic DNA)

ANTI-SENSE:NO

SEQUENCE DESCRIPTION:SEQ ID NO:30:

ACATGCTGGG TGGATCCGGA TC 22

SEQ ID NO:31

LENGTH:20 bases

TYPE:nucleic acid

STRANDEDNESS:single

TOPOLOGY:linear

MOLECULE TYPE:Other nucleic acid (synthetic DNA)

ANTI-SENSE:YES

SEQUENCE DESCRIPTION:SEQ ID NO:31:

GGAACTGTCT CAAACCTTAG 20

SEQ ID NO:32

LENGTH:28 bases

TYPE:nucleic acid

STRANDEDNESS:single

TOPOLOGY:linear

MOLECULE TYPE:Other nucleic acid (synthetic DNA)

ANTI-SENSE:YES

SEQUENCE DESCRIPTION:SEQ ID NO:32:

GTTTAAGTTT GTGGGTCATG AATTCTCC 28

## Claims

1. An isolated and non-naturally occurring polypeptide having calmodulin-binding activity and actin-binding activity and comprising the amino acid sequence shown in SEQ ID No 1 in a polypeptide molecule.

2. An isolated and non-naturally occurring polypeptide according to claim 1 having the amino acid sequence shown in SEQ ID No. 1, SEQ ID No 2, SEQ ID No. 3 or SEQ ID No. 4.

3. An isolated gene which codes for a polypeptide according to claim 1 or 2.

4. An isolated gene according to claim 3 comprising the DNA sequence shown in SEQ ID No. 7 in a gene molecule.

5. An isolated gene according to claim 4 having the DNA sequence shown in SEQ ID No. 7, SEQ ID No. 8, SEQ ID No. 9 or SEQ ID No. 10.

6. An isolated gene according to claim 3 which can hybridise the gene of claim 4 or claim 5 under stringent conditions.

## Patentansprüche

1. Isoliertes und nicht-natürlich vorkommendes Polypeptid mit Calmodulin-Bindungsaktivität und Actin-Bindungsaktivität, welches die in SEQ ID Nr. 1 gezeigte Aminosäuresequenz in einem Polypeptidmolekül umfaßt.

2. Isoliertes und nicht-natürlich vorkommendes Polypeptid nach Anspruch 1, welches die in SEQ ID Nr. 1, SEQ ID Nr. 2, SEQ ID Nr. 3 oder SEQ ID Nr. 4 gezeigte Aminosäuresequenz aufweist.

3. Isoliertes Gen, weiches für ein Polypeptid nach Anspruch 1 oder 2 kodiert.

4. Isoliertes Gen nach Anspruch 3, welches die in SEQ ID Nr. 7 gezeigte DNA-Sequenz in einem Genmolekül umfaßt.

5. Isoliertes Gen nach Anspruch 4, welches die in SEQ ID Nr. 7, SEQ ID Nr. 8, SEQ ID Nr. 9 oder SEQ ID Nr. 10 gezeigte DNA-Sequenz aufweist.

6. Isoliertes Gen nach Anspruch 3, welches das Gen von Anspruch 4 oder 5 unter stringenten Bedingungen hybridisieren kann.

## Revendications

1. Polypeptide isolé et n'existant pas à l'état naturel, ayant une activité de liaison à la calmoduline et une activité de liaison à l'actine et comprenant la séquence d'amino-acides représentée dans la SEQ ID N° 1 dans une molécule du polypeptide.

2. Polypeptide isolé et n'existant pas à l'état naturel suivant la revendication 1, ayant la séquence d'amino-acides représentée dans la SEQ ID N° 1, la SEQ ID N° 2, la SEQ ID N° 3 ou la SEQ ID N° 4.

3. Gène isolé qui code pour un polypeptide suivant la revendication 1 ou 2.

4. Gène isolé suivant la revendication 3, comprenant la séquence d'ADN représentée dans la SEQ ID N° 7 dans une molécule du gène.

5. Gène isolé suivant la revendication 4, ayant la séquence d'ADN représentée dans la SEQ ID N° 7, la SEQ ID N° 8, la SEQ ID N° 9 ou la SEQ N° 10.

6. Gène isolé suivant la revendication 3, qui peut s'hybrider au gène suivant la revendication 4 ou la revendication 5 dans des conditions drastiques.
